# EUROPEAN PATENT SPECIFICATION

(11) **EP 2 258 463 B1**
(45) Date of publication and mention of the grant of the patent: **13.11.2013**
(21) Application number: 09720493.7
(22) Date of filing: 09.03.2009
(51) Int. Cl.: B01D 53/62, B01D 53/14, B01D 53/96, C12N 1/12, C12F 3/02

(54) **LIQUID-PHASE GAS COLLECTION**
FLÜSSIGPHASEN-GASSAMMLUNG
CAPTURE DE GAZ EN PHASE LIQUIDE

(30) Priority: 14.03.2008 ES 200800753
(43) Date of publication of application: 08.12.2010
(73) Proprietor: Endesa Generación, S.A., 41004 Sevilla (ES)
(72) Inventor: ACIÉN FERNÁNDEZ, Francisco Gabriel, E-41004 Sevilla (ES); MOLINA GRIMA, Emilio, E-41004 Sevilla (ES); FERNÁNDEZ SEVILLA, José María, E-41004 Sevilla (ES); GONZÁLEZ LÓPEZ, Cynthia Victoria, E-41004 Sevilla (ES); LLAMAS MOYA, Bernardo, E-41004 Sevilla (ES); BALLESTEROS APARICIO, Juan Carlos, E-41004 Sevilla (ES)
(74) Representative: Pons Ariño, Angel
(86) International application number: PCT/ES2009/070058
(87) International publication number: WO 2009/112624

(56) References cited:
- WO-A1-92/10270
- ES-A1- 2 262 432
- ES-B1- 2 262 432
- ES-T3- 2 062 883
- US-A- 3 856 921
- US-A1- 2003 073 231
- NYSING, R.A.T.O.; KRAMERS, H.: "ABSORPTION OF CO2 IN CARBONATE BICARBONATE BUFFER SOLUTIONS IN A WETTED WALL COLUMN", CHEMICAL ENGINEERING SCIENCE, vol. 8, no. 1-2, 4 April 1958 (1958-04-04) , pages 81-89, XP002631444,
- KLOOSTERMAN ERNST G ET AL: "Influence of ionic strength on the absorption of carbon dioxide in carbonate/bicarbonate buffer solutions", INDUSTRIAL AND ENGINEERING CHEMISTRY RESEARCH, vol. 26, no. 11, November 1987 (1987-11), pages 2216-2222, XP002631445,
- KLOOSTERMAN, E. ET AL.: 'Influence of Ionic Strength on the Absorption of C02 in Carbonate/ Bicarbonate Buffer Solutions' IND. ENG. CHEM. RES. vol. 26, 1987, pages 2216 - 2222
- BEDEKAR, S.: 'Properties of sodium carbonate- bicarbonate solutions' JOURNAL OF APPLIED CHEMISTRY. vol. 5, no. ISS.5, 1955, pages 72 - 75

## Description

This invention falls within the field of chemistry, chemical engineering and the environment. It relates to a gas capture process in the liquid phase, and the subsequent recovery of this phase, by coupling the process to a biological system.

### PRIOR STATE OF THE ART

The increase in the emission of pollutant gases, mostly generated by combustion processes, particularly of fossil fuels (coal, oil, natural gas), may lead to an overheating of the earth's surface (greenhouse effect). This represents an economic and environmental problem for developed countries, but especially for developing countries, whose high level of CO₂ emissions may entail significant sanctions at the international level, thereby delaying their incorporation into First-World countries.

The solution may be addressed either by developing new, less polluting technologies, or by developing systems and processes capable of capturing those gas emissions and, if possible, reconverting them. CO₂, which is the main gas that causes the greenhouse effect, is involved in the carbon cycle, where the photosynthesis process acts as a natural drain, allowing for the conversion thereof into organic matter, and maintaining the atmospheric levels of this gas (0.03% v/v). However, this transformation process from inorganic to organic carbon, which occurs naturally, is very slow.

Numerous systems have been developed for the capture of CO₂, and other gases such as NOₓ or SOₓ, which may be classified into physical adsorption systems, separation using membranes, cryogenic distillation and chemical absorption systems.

Physical adsorption systems use materials capable of absorbing CO₂, which is later "desorbed" through changes in T or P (pressure and temperature swing adsorption, PTSA). Absorbent materials include: active coal, mesoporous materials, zeolites, aluminas and hydrotalcites. The disadvantages are that they require a temperature increase in the desorption phase (50°C-100°C), the low adsorption capacity per unit bed volume, and that, subsequently, the material is either discarded or desorbed, such that the CO₂ is re-incorporated into the atmosphere.

The selectivity of membranes toward different gases is related to the material whereof they are manufactured (polymers, metals, ceramic materials). The separation is improved by means of high-pressure streams. The advantage of using membranes lies in that they improve the mass transfer area and eliminate the problems associated with gas-liquid contact. In this application, the membrane does not perform the separation operation; instead, the latter is performed with ethanolamines. In order to prevent the destruction or deterioration of the membrane, the latter must be made of Polytetrafluoroethylene (PTFE). Use of this technology reduces the process installation and operating costs by 30%-40%, regardless of the improvements that may be introduced in the use of ethanolamines (Herzog H., Falk-Pedersen O., 2000, The Kvaerner membrane contactor: lessons from a case study in how to reduce capture costs. Fifth International Conference on Greenhouse Gas Control Technologies, Cairns, Australia: 121-5). The disadvantage is that as yet they have not been used at the scale and under the conditions, in terms of availability and cost, which are necessary for CO₂ capture systems (and the CO₂ recovery phase).

Cryogenic distillation takes place by means of a number of compression, cooling and expansion steps, whereafter the gas components may be separated in a distillation column. This technology is primarily used to separate the impurities in a high-purity CO₂ stream. The disadvantage is that as yet it has not been used at the scale and under the conditions, in terms of availability and cost, which are necessary for CO₂ capture systems in large emission foci.

Most chemical absorption processes are based on potassium carbonate or alkanolamines. Monoethanolamine (MEA) and diethanolamine (DEA) are stronger alkalis than potassium carbonate; for this reason, they reduce the acid gas content to a larger extent. The absorption capacity is also greater for alkanolamines. Additives are habitually used to increase the absorption capacity of K₂CO₃ solutions, and they have the disadvantage of a lower gas purification efficacy.

**Table 1 summarises the different chemical absorption processes:**

| **PROCESSES** | | **ABSORBENT (in aqueous solution)** |
|---|---|---|
| **USING AMINES** | Adip (Shell Dev. Co.) | Diisopropanolamine |
| | Amine Guard (Union Carbide) | MEA + stream inhibitors |
| | DEA | Diethanolamine |
| | Econamine (Fluor Corp.) | Diethanolamine |
| **USING CARBONATES** | Benfield (Union Carbabide) | K₂CO₃ + additives |
| | Catacarb (Eickmeyer & Ass.) | K₂CO₃ + additives |
| | Gianmarco-Vetrocoke (Gianmarco.) | K₂CO₃ + additives |

The main disadvantages of CO₂ capture systems in the liquid phase which use ethanolamines are that a lot of thermal energy is required in order to regenerate the MEA or DEA solution, due to its affinity for acids. Amines may also lead to undesirable chemical reactions (especially methanolamine with COS and CS₂), to form products that degrade the solution and increase corrosion.

Therefore, the most widely used process nowadays is absorption in an ethanolamine-enriched aqueous phase. This is an absorption process that includes a chemical reaction which makes it possible to achieve much greater CO₂ capture efficiencies than those achieved by physical absorption, without a chemical reaction, wherein only concentrations equal to the solubility of CO₂ in the corresponding medium (10⁻⁵ mol CO₂/l, 0.5 mg CO₂/l) may be reached. However, it has serious environmental disadvantages, since it is toxic or harmful, and may not be easily regenerated. The process consists of having the gas stream to be purified pass through an absorption tower irrigated with ethanolamine solutions, such that the CO₂ reacts with said ethanolamines and is retained in the solution. The solution containing the retained carbon dioxide is subsequently passed through another tower, in this case a desorption tower, where the CO₂ is desorbed by means of a heat supply, and the CO₂ may already be compressed or managed as a separate compound. Thus, an ethanolamine solution is regenerated, which is re-circulated to the absorption column. Despite the corrosion and contamination problems explained above, this process has been applied to the capture of CO₂ contained in combustion gases, and there are commercial process based on this technology for the capture of the CO₂ contained in combustion gases, and currently they represent the only technological alternative available (Herzog H., 2001, What future for carbon capture and sequestration? Environmental Science Technology, 35(7): 148A-53A).

In order to improve the efficacy of ethanolamine-based processes, modifications have been proposed that use other absorbents (diethanolamines, mixtures of ethanolamines, additives, KS-1, etc.) which improve the absorption capacity of the liquid phase, and the use of structured filler columns that improve the transfer capacity, thereby reducing power consumption and the size of the equipment (Yagi Y., Mimura T., lijima M., Yoshiyama R., Kamijo T., Yonekawa T., 2004, Improvements of carbon dioxide capture technology from flue gas. GHGT, International Conference on Greenhouse Gas Control Technology, Tokyo, 2003). Despite these improvements, the process' energy consumption is greater than 3.3 MJ/kg CO₂, as compared to 4-5 MJ/kg CO₂ for non-optimised processes (Gambini M. and Vellini M., 2000, CO2 emission abatement from fossil fuel power plants by exhaust gas treatment. Proceedings of 2000 International Joint Power Generation Conference Miami Beach, Florida, July 23-26, 2000).

The most widely-spread alternative process is the use of concentrated carbonate solutions, primarily potassium carbonate solutions, which, in a first step, absorb CO₂ by means of a chemical reaction, in order to be subsequently regenerated by means of a heat supply, CO₂ being released in the form of a pure gas stream. This process is commercially called Benfield process. The solubility and the reaction of CO₂ in concentrated potassium carbonate solutions has been extensively studied (Benson, H.E., Field, J.H., Jimeson, R.M., 1954. CO2 absorption employing hot potassium carbonate solutions. Chemical Engineering Progress 50 (7), 356-364; Tosh, J.S., Field, J.H., Benson, H.E., Haynes, W.P., 1959. Equilibrium study of the system potassium carbonate, potassium bicarbonate, carbon dioxide, and water. United States Bureau of Mines, 5484). These studies indicate that potassium carbonate has a lower regeneration heat than ethanolamines, but the reaction rate thereof is slower than that of ethanolamines. The Benfield process uses potassium carbonate concentrations of about 20% by weight (200 g K₂CO₃/l). The use of these high concentrations of alkaline carbonates has also been proposed for the precipitation of pollutant compounds such as nitrogen and sulfur oxides in combustion gases, by means of "scrubbing" (US 5100633 A). In this case, the consumption of alkali is equivalent to the quantity of pollutants to be removed, the latter being obtained in the form of a precipitate that may be subsequently used in the manufacturing of cement. Another modification based on alkaline carbonates is using diluted solutions of said salts, with an initial pH of 8, which, after being placed in contact with combustion gases, absorb CO₂ and reduce the pH thereof to values of 4, the resulting liquid phase being poured into large masses of water, following dilution, if necessary (US 6890497 B2). In all these cases, the carbonate directly reacts with CO₂, to produce bicarbonate, with significant pH variations taking place and working at high carbonate concentrations, or at very low concentrations such that the resulting product may be poured into the natural environment. On the other hand, a thermal regeneration of the aqueous phase is only performed in the Benfield process, whereas, in the rest of the processes, the aqueous phase is not regenerated, but, instead, as much mineral carbonate is added as has been previously consumed or it is poured into the natural environment without being regenerated.

WO 92/10270 discloses a process for capturing pollutants such as CO₂ from a combustion gas comprising placing a liquid phase in contact with the combustion gas stream, wherein the liquid phase contains carbonate-bicarbonate buffer salts with a total concentration of inorganic carbon less than or equal to 25 g/I and pH values ranging between 8 and 9, and recovering a CO₂-enriched liquid phase. The influence of the carbonate-bicarbonate ratio on absorption of CO₂ in carbonate-bicarbonate buffer solutions is disclosed for example in Nysing, R.A.T.O., Kramers, H., Absorption of CO2 in carbonate bicarbonate buffer solutions in a wetted wall columns, Chemical Engineering Science, vol. 8, no. 1-2, 4 April 1958, pages 81-89.

Since the other alternatives to CO₂ chemical absorption processes (such as separation using membranes, cryogenic fractionation or adsorption using molecular sieves) are less efficient both in terms of energy and economically (Herzog H., 1999, An introduction to CO2 separation and capture technologies. Energy Laboratory Working Paper. Massachusetts Institute of Technology, Cambridge), it is necessary to develop new processes capable of accelerating the capture of CO₂, which facilitate the subsequent regeneration thereof. Different groups and entities worldwide are conducting studies which analyse oxycombustion, CO₂ fixation by forest masses, the retention thereof in the sea and the photosynthetic fixation of CO₂. In this regard, photosynthetic microorganisms, such as microalgae or cyanobacteria, are unique and very valuable, since they are the greatest converters of CO₂ into O₂ in the planet, with carbon fixation yields that are over five times greater than corn plantations, in addition to being the main source of biomass and one of the most variable ecological groups in the world (Pulz O., 2001, Photobioreactors: production systems for phototrophic microorganisms). The use of some microorganisms for these purposes has been patented (WO 2006/120278). However, the necessary concentrations to optimise this process are not specified and, as in other patents, it is based on the direct injection of the combustion gases. This entails numerous adaptation problems for the organisms and, if the reactor is an open reactor, the possibility of CO₂ release (desorption of the dissolved CO₂), which reduces the system's benefits.

However, more or less regardless of the organism used, it is necessary to develop a process that is capable of absorbing CO₂ at high concentrations and at a much higher rate than occurs naturally, and that is susceptible to being coupled to a biological system that allows for the conversion of this absorbed CO₂ into organic matter. This would allow for the conversion of CO₂, primarily resulting from combustion, into organic matter.

### EXPLANATION OF THE INVENTION

There is a need to find a system that allows for the conversion of the carbon resulting from CO₂ into organic matter, at a higher rate than under natural conditions. The authors of this invention have managed to establish the reaction conditions necessary to couple a capture process of CO₂ and other gases, in the liquid phase, primarily arising from combustion processes, to a biological system that allows for the conversion thereof into organic matter.

This process captures concentrations of CO₂, and other gases, similar to those of other processes (ethanolamines, Benfield), with a lower energy consumption and without the need for treatments at different pressures and/or temperatures, additionally allowing for the conversion of these combustion gases, in particular the inorganic carbon in CO₂. What is essential is to achieve adequate pH ranges, which are compatible with the concentrations of inorganic carbon, sodium carbonate and bicarbonate necessary for the absorption of high concentrations of CO₂, and compatible with the adequate culture conditions for the photosynthetic microorganisms. Therefore, it entails two differentiated steps that are optimised separately, albeit taking into consideration the necessary coupling between them.

Therefore, the process is based on the buffer capacity of the carbonate and bicarbonate solutions, which makes it possible to reconcile moderately high concentrations of inorganic carbon with the suitable pH ranges to incorporate the liquid phase into a culture of photosynthetic organisms.

The process according to the invention is defined in claim 1

In a preferred embodiment of this aspect of the invention, the concentration of inorganic carbon has values ranging between 2 and 12 g/l. In another more preferred embodiment of this aspect of the invention, the inorganic carbon concentration values range between 3 and 8 g/l. In a particular embodiment, the concentration of inorganic carbon is approximately 4 g/l.

In a more preferred embodiment of this aspect of the invention, the pH values range between 9 and 11. In a particular embodiment of the invention, the pH values are about 10.

The concentrations of pH may be achieved, for example, by adding NaHCO₃ in concentrations ranging between 5 and 22 g/l and Na₂CO₃ in concentrations ranging between 10 and 44 g/l to the liquid phase. In a particular embodiment of the invention, the concentrations were 11 g/l of NaHCO₃ and 22 g/l of Na₂CO₃. Under these conditions, a CO₂ purification efficiency of 80% may be obtained, as well as purification efficiencies of 50% and 95% for NOₓ and SOₓ, respectively. The composition of the aqueous phase may be modified by increasing the Na₂CO₃ content, which increases the pH and improves the CO₂ capture efficacy, albeit with the disadvantage that subsequent regeneration of the aqueous phase by means of biological treatment is hindered. The use of carbonate-bicarbonate-enriched aqueous phases makes it possible to obtain gas purification yields analogous to those obtained using ethanolamine-enriched aqueous phases, albeit without the energy consumptions necessary to regenerate them. Moreover, it is necessary to work with lower carbonate and bicarbonate concentrations than the solubility product constant of the precipitated salts which might be achieved with the cations present in the medium.

High concentrations of inorganic carbon would reduce the algae productivity, but very low concentrations would reduce the capture of CO₂. The pH must be greater than 8, because, otherwise, the carbonate-bicarbonate buffer would not exert its buffering action, and absorption of significant quantities of CO₂ would not take place, but it must be lower than 12, in order to allow for the subsequent regeneration thereof. The classic "scrubber" process maintain pH values greater than 12, which makes it impossible to couple them to biological systems of photosynthetic organisms.

Contact between the gas phase to be purified and the liquid phase may be performed by any commonly used technique, such as ordered or structured random-packed columns, spray columns, step columns or fluidisation columns. It is preferred to use the latter, since they provide the highest purification yields with the lowest energy consumption and required liquid phase volume. In this case, the recommended residence time for the aqueous phase ranges between 15-30 minutes. Thus, a 100-I fluidisation column is sufficient to purify a 200-I/min gas stream, capturing 80% of the CO₂ contained therein, by the circulation of 4 l/min of carbonate-bicarbonate-enriched aqueous phase. This process is compatible with the use of fresh water and sea or brackish water, and even waters from agricultural uses or secondary wastewater treatment, in the preparation of the carbonate-bicarbonate-enriched aqueous phase, which entails an additional environmental and economic advantage.

The gas stream preferably results, without being limited thereto, from fossil fuel combustion processes (coal, oil, natural gas). These combustion processes mostly arise from stationary combustion sources, such as incinerators, cement factories, metallurgical industry facilities and, in particular, electricity generation plants.

In general, in order to have a sufficient buffer capacity, thereby increasing the capture of CO₂ whilst maintaining adequate pH ranges, the carbonate:bicarbonate ratio in the liquid phase is 2:1. The reaction that would take place is summarised as follows:

CO₃²⁻ + H₂O → HCO₃⁻ + OH⁻

OH- + CO₂ → HCO₃⁻

CO₃²⁻ + CO₂ → 2 HCO₃⁻

This CO₂ capture phase is prior to, and is performed separately from, the coupling phase to the biological system, which allows for the optimisation of both processes, thereby obtaining a higher yield. However, the conditions for both processes (pH, temperature, salt and ion concentrations, etc.) must be the adequate ones in order to be integrated into a single process.

Unlike the existing CO₂-fixation processes that use microalgae or cyanobacteria found in the state of the art, in the process of the invention, the CO₂ is supplied to the reactor previously dissolved in the aqueous phase, in the different water-soluble carbonated forms (CO₂, H₂CO₃, HCO₃⁻, CO₃⁼), for which reason the transport efficiency is 100%. As regards the photosynthetic organisms that may be used, preferably, without being limited thereto, both cyanobacteria (Bacteria; Cyanobacteria) and green algae (Eukaryota; Viridiplante; Chlorophyta) may be used, with the requirement that the microorganism to be used may bear alkaline pH values, as well as high total inorganic carbon concentrations. Cyanobacteria are preferably used, due to the lower risk of contamination, atmospheric nitrogen fixation capacity, greater tolerance to high pH and high concentrations of inorganic carbon, and ease of sedimentation and harvesting. For example, species of *Spirulina* and *Anabaena* would meet these requirements and give good results.

As regards the culture system, both open and closed reactors may be used, although open reactors are preferably used due to the lower cost thereof, or low-cost vertical flat-plate reactors. Preferably, using vertical flat-plate reactors made of flexible materials. The required bioreactor surface area is a function of the biological purification yield, which depends on the microorganism, the reactor design and the operating conditions thereof.

Another aspect relates to the process of the invention for the purification of gases arising from the combustion of fossil fuels. Said gases may come from sectors as diverse as transport, household consumption or, more preferably, industrial combustion processes. Gases with high proportions of CO₂ may come from electricity generation plants, cement factories or any other industrial plants that develop CO₂-generating processes. Another aspect relates to the process of the invention for the purification of gases from electricity generation plants.

The microalgae, cyanobacteria or other photosynthetic organisms in the photobioreactor may use the CO₂, which has been previously captured by the liquid phase, to produce biomass. In order to maintain optimal levels of the photosynthetic organisms inside the photobioreactors, the biomass is periodically extracted therefrom.

Optionally, the extracted biomass may be dessicated, and at least a portion of the water contained therein is evaporated. This water may be subsequently condensated, and re-utilised to prepare the liquid phase that will be used in the CO₂ capture step.

The biomass recovered from the photobioreactor may be directly used as solid fuel or converted, for example, into biodiesel or another organic fuel, by means of pyrolysis and/or thermochemical liquefaction processes. These processes are well-known in the state of the art, and are described, for example, in Yutaka et al., 1994 (Recovery of liquid fuel from hydrocarbon-rich microalgae by thermochemical liquefaction," Fuel 73(12): 1855-1857). The biomass may also be used for gasification, to produce flammable organic gases that may be used in energy plants.

In this specification, "combustion gases" are understood to mean gases that are primarily generated by fossil fuel combustion processes (coal, oil, natural gas). They primarily refer to CO₂, but also, without being limited thereto, to NOₓ nitrogen oxides (nitrogen monoxide and dioxide, NO and NO₂, respectively) and sulfur oxides or SOₓ (sulfur dioxide and trioxide, SO₂ and SO₃).

The solution of carbonate-bicarbonate buffer salts "in a 2:1 ratio" means that the salt concentrations are adequate to capture the combustion gases, primarily CO₂, from the gas stream, and that the buffer capacity is sufficient to maintain an adequate pH that allows for the incorporation of the gas-enriched liquid phase into a biological system. As has been described in this specification, this adequate ratio is obtained by adding soluble carbonate and bicarbonate salts, in this case, sodium carbonate and bicarbonate, in concentrations ranging between 5 and 22 g/l for NaHCO₃ and between 10 and 44 g/l for Na₂CO₃.

In this specification, "photobioreactor" is understood to mean a bioreactor that is dependent on sunlight. As understood in this specification, a "bioreactor" is a container or system that maintains a biologically active environment. Therefore, the photobioreactor is a container wherein a process takes places that involves photosynthetic organisms and seeks to maintain certain favourable environmental conditions (pH, temperature, oxygen concentration, etc.) for the element being cultured. The photobioreactor contains, or is configured to contain, a liquid medium that comprises at least one photosynthetic organism species and has, either a light source capable of stimulating photosynthesis, or a surface with at least a portion that is transparent to light with a wavelength capable of stimulating photosynthesis (for example, light with a wavelength ranging between 400 and 700 nm).

The term "photosynthetic organisms" or "biomass", as used in this specification, includes all organisms capable of performing photosynthesis, such as plants (or the cells thereof) in the broadest sense, algae (including, without being limited thereto, those belonging to the taxonomic categories *Chlorophyta, Rhodophyta* and *Phaeophyta*) and cyanobacteria, or other photosynthetic bacteria, in unicellular or multicellular forms, which are capable of growing in a liquid phase (except when the term "biomass" is used in other documents incorporated into this specification, in which case it may be used in a broader sense, also including organic matter from a large variety of plants and/or animals). The term also includes artificially modified or genetically-engineered organisms.

In this specification, "green algae" is understood to mean all those organisms that may be classified within the Phylum *Chlorophyta.* The term "microalga", as used herein, refers to a single-cell photosynthetic organism, preferably of the Phylum *Chlorophyta.* The term "cyanobacteria" includes all the species of the Phylum *Cyanobacteria.*

Throughout the description and the claims, the word "comprises" and the variants thereof are not intended to exclude other technical characteristics, additives, components or steps. For those skilled in the art, other objects, advantages and characteristics of the invention will arise partly from the description and partly from the practise of the invention. The following examples and drawings are provided for illustrative purposes, and are not intended to limit the scope of this invention.

### BRIEF DESCRIPTION OF THE FIGURES

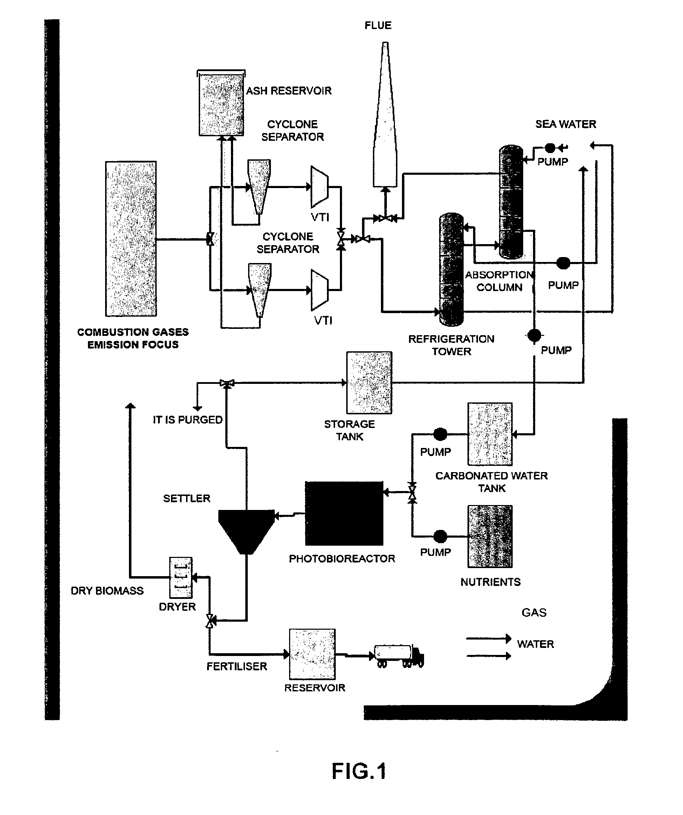

Figure 1 shows a possible flow diagram of the process of the invention, by means of the capture of pollutants in the aqueous phase by absorption with a chemical reaction, and subsequent regeneration of the aqueous phase by biological treatment with photosynthetic microorganisms in photobioreactors.
Figure 2 shows the variation of the purification of SO₂ and NOₓ with the concentration of the compound in the inlet gas and the liquid/gas ratio used.

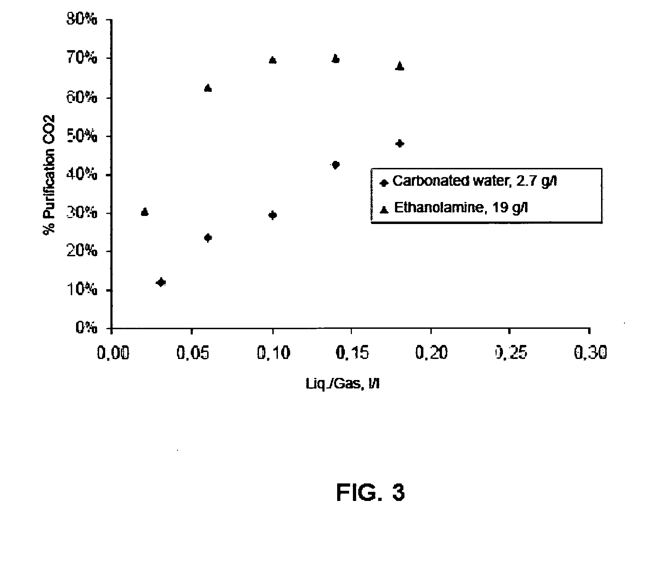

Figure 3 shows a comparison of the CO₂ purification efficacy using carbonated solutions or ethanolamine solutions as a function of the operating conditions.

### DETAILED DESCRIPTION OF THE EMBODIMENTS

Below we will illustrate the invention with assays performed by the inventors, which show the effectiveness of the process of capturing CO₂ from combustion processes and the regeneration thereof by means of a biological system.

### EXAMPLE

In this example, the inventors have designed a demonstration plant that makes it possible to assay the process of the invention, with a 250-I fluidisation column, for the purification of gases from a gas-oil boiler.

### CO₂ capture system

The capture of CO₂ was performed in a 250-I fluidisation column, humidifying the gas stream with the liquid phase. The system bears inundation flow rates of 20 I/min of liquid and 350 I/min of gases, the minimum flow rate to wet the column being 4 I/min of liquid (experimental data).

The liquid phase was prepared by adding about 11 g/l of NaHCO₃ and 22 g/I of Na₂CO₃, reaching a pH of 10. The total concentration of inorganic carbon was 4.0 g/l.

Under these conditions, a CO₂ purification efficiency of 80% may be achieved, as well as purification efficiencies of 50% and 95% for NOₓ and SOₓ, respectively.

Operating with this column, with 10% binary air-CO₂ mixtures as the gas phase and water from the mains as the liquid phase, the mass transfer volumetric coefficient was determined. The results show that the value of said coefficient is high, reaching values of 50 I/h for K_{L}a_{L}. Furthermore, when operating without adding an alkaline phase, purification efficiencies of 98% are reached, albeit only in the case of very low gas flow rates, of 4 l/min, and high liquid flow rates. This is due to the alkalinity of the water, which is sufficient to purify small gas flow rates, but not high net CO₂ absorption flow rates.

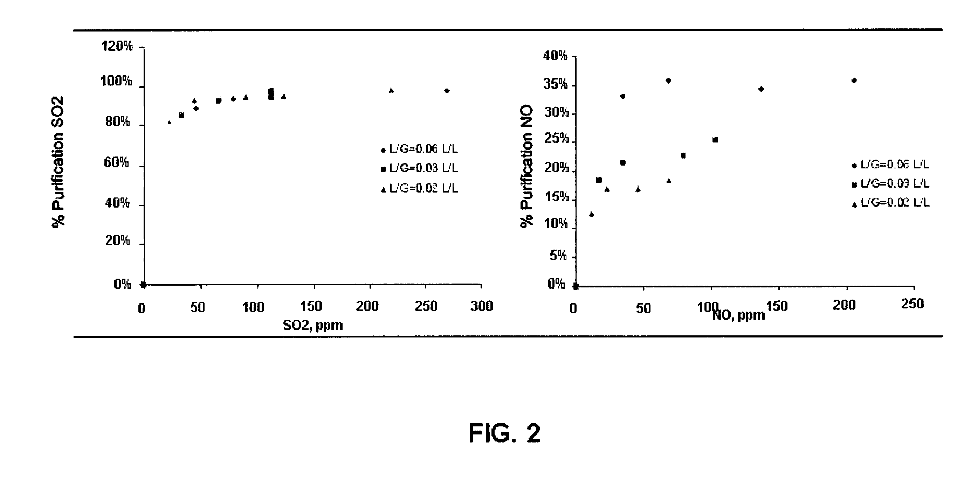

The system has been assayed with gas mixtures containing SO₂ (above 50 ppm) and NOx. The results show that SO₂ is a very acid gas that tends to be very effectively absorbed in alkaline phases. As regards NOx, its acid character is much less marked, for which reason its mass transfer rate is lower. Fig. 2 shows the variation of the purification of SO₂ and NO with the concentration of the compound in the inlet gas and the liquid/gas ratio used.

### Comparison of the CO₂ capture system using carbonated solutions or ethanolamine solutions

The behaviour observed confirms that the chemical reaction of ethanolamines with CO₂ is faster than with carbonate; therefore, the size of the equipment and the required flow rates are reduced, although the use of bicarbonate-carbonate-enriched aqueous phases exhibits yields within a similar order of magnitude; for this reason, it may be feasible for some lower-scale applications. Moreover, it is environmentally compatible, whereas it is not possible to use ethanolamines for coupling with biological systems.

The advantages of this capture system may be summarised as follows:
1. CO₂ capture yields similar to those obtained with chemical absorption with amines.
2. The compound used as the absorbent is cheaper and more readily available.
3. The compound used as the absorbent is not toxic or harmful.
4. It allows for the use of any quality of water.
5. De-sulfurization, reduction of NOx and CO₂ capture take place in a single system.
6. Conversion and direct use of the CO₂, or as fuel (biomass, bioethanol, etc.) the CO₂ emissions whereof are neutral.
7. Use of a biological regeneration system.

### Biological regeneration system

The photosynthesising microorganisms may be, preferably, of two types: cyanobacteria and algae (preferably microalgae). Depending on the microorganism and the culture conditions, different products may be obtained.

Assays have been performed with *Anabaena, Synechocystis, Chlorococcum, Botryococcus, Spirulina* and *Chlorella.* Those which *a priori* have exhibited the best behaviour are *Synechocysitis* and *Chlorococcum. Botryococcus* has exhibited a low growth rate, although it is interesting due to its high lipid content. On the other hand, *Chlorella* has shown a correct growth, as has *Spirulina,* although *Spirulina* has the advantage of being perfectly adapted to carbonate-rich media and high pH.

The assays have been performed in flat-plate photobioreactors under laboratory conditions, albeit simulating outdoor conditions by operating within the solar cycle, using the liquid phase resulting from the gas absorption phase, after using it for the purification of real combustion gas from a gas-oil boiler, as the culture medium. Due to the use of artificial lighting, the maximum photosynthetic radiation used was 850 µE/m²s, which is three times lower than the maximum value under outdoor conditions.

Using 300-I vertical flat-plate reactors made of flexible materials, and using the cyanobacterium *Anabaena marina,* CO₂ fixation rates of 100 g/m² day have been achieved. According to these results, in order to regenerate the liquid phase produced in the capture of the CO₂ contained in a 200-I/min gas stream, 120 m² of surface area occupied by flat-plate reactors as those described above would be required. The required surface area is a function of the biological purification yield, which depends on the microorganism, the reactor design and the operating conditions thereof.

## Claims

1. Process for capturing CO₂ and optionally NOₓ and/or SOₓ from a combustion gas which process comprises:
a. Placing a liquid phase in contact with the combustion gas, wherein the liquid phase contains carbonate-bicarbonate buffer salts in a 2:1 ratio, calculated on the basis g/l with a total concentration of inorganic carbon less than or equal to 25 g/l and pH values ranging between 8 and 12,
b. recovering the CO₂-enriched liquid phase obtained in step a) and regenerating it in a photobioreactor.

2. Process according to the preceding claim, wherein the concentration of inorganic carbon has values ranging between 2 and 12 g/l.

3. Process according to claim 1, wherein the concentration of inorganic carbon has values ranging between 3 and 6 g/l.

4. Process according to any of the preceding claims, wherein the pH has values ranging between 9 and 11.

5. Process according to the preceding claim, wherein the photobioreactor is a flat-plate bioreactor.

6. Process according to any of preceding claims 5-6, wherein the photosynthetic organism in the photobioreactor is a cyanobacterium or a green alga.

7. Process according to claim 6, wherein the photosynthetic organism is a cyanobacterium.

8. Process according to any of the preceding claims, wherein the pollutant gases are obtained from the combustion of fossil fuels.

9. Process according to any of claims 1-7, wherein the pollutant gases come from electrical energy generation plants.

## Patentansprüche

1. Verfahren zum Einfangen von CO₂ und wahlweise von NOₓ und/oder SOₓ aus einem Verbrennungsgas, wobei das Verfahren folgendes umfasst:
a. Kontaktieren einer Flüssigphase mit dem Verbrennungsgas, wobei die Flüssigphase Karbonat-Bikarbonat-Puffersalze im Verhältnis 2:1, berechnet als g/l, enthält, mit einer Gesamtkonzentration an anorganischem Kohlenstoff kleiner oder gleich 25 g/l und pH-Werten im Bereich von 8 bis 12,
b. Rückgewinnung der im Schritt a) erhaltenen, an CO₂ angereicherten Flüssigphase und deren Regenerierung in einem Photobioreaktor.

2. Verfahren gemäß dem vorhergehenden Anspruch, wobei die Konzentration an anorganischem Kohlenstoff Werte zwischen 2 und 12 g/l aufweist.

3. Verfahren gemäß Anspruch 1, wobei die Konzentration an anorganischem Kohlenstoff Werte zwischen 3 und 6 g/l aufweist.

4. Verfahren gemäß einem der vorhergehenden Ansprüchen, wobei der pH Werte zwischen 9 und 11 aufweist.

5. Verfahren gemäß dem vorhergehenden Anspruch, wobei der Photobioreaktor ein Platten-Bioreaktor ist.

6. Verfahren gemäß einem der vorhergehenden Ansprüche 5 bis 6, wobei der photosynthetische Organismus im Photobioreaktor ein Cyanobakterium oder eine Grünalge ist.

7. Verfahren gemäß Anspruch 6, wobei der photosynthetische Organismus ein Cyanobakterium ist.

8. Verfahren gemäß einem der vorangehenden Ansprüche, wobei die Schadgase aus der Verbrennung fossiler Brennstoffe gewonnen werden.

9. Verfahren gemäß einem der Ansprüche 1 bis 7, wobei die Schadgase von Stromerzeugungswerken stammen.

## Revendications

1. Procédé pour capturer le CO₂ et éventuellement le NOₓ et/ou SOₓ d'un gaz de combustion, ledit procédé consiste en :
a. La mise en contact d'une phase liquide avec le gaz de combustion, où la phase liquide contient des sels tampons de carbonate-bicarbonate dans une proportion de 2:1, calculée sur le g/e de base avec une concentration totale de carbone inorganique inférieure ou égale à 25 g/l et des valeurs de pH comprises entre 8 et 12,
b. la récupération de la phase liquide enrichie en CO₂ obtenue au cours de l'étape a) et sa régénération dans un photobioréacteur.

2. Procédé selon la revendication précédente, dans lequel les valeurs de concentration de carbone inorganique sont comprises entre 2 et 12 g/l.

3. Procédé selon la revendication 1, dans lequel les valeurs de concentration de carbone inorganique sont comprises entre 3 et 6 g/l.

4. Procédé selon n'importe laquelle des revendications précédentes, dans lequel les valeurs de pH sont comprises entre 9 et 11.

5. Procédé selon la revendication précédente, dans lequel le photobioréacteur est un bioréacteur plat.

6. Procédé selon n'importe laquelle des revendications précédentes 5-6, dans lequel l'organisme photosynthétique dans le photobioréacteur est une cyanobactérie ou une algue verte.

7. Procédé selon la revendication 6, dans lequel l'organisme photosynthétique est une cyanobactérie.

8. Procédé selon n'importe laquelle des revendications précédentes, dans lequel les gaz polluants sont obtenus à partir de la combustion de combustibles fossiles.

9. Procédé selon n'importe laquelle des revendications 1-7, dans lequel les gaz polluants proviennent de centrales de production d'énergie électrique.
